Europäisches Patentamt

**European Patent Office**

Office européen des brevets

(19)

(11) Publication number: **0 178 050**
A1

## (12) EUROPEAN PATENT APPLICATION

(21) Application number: 85305744.6

(22) Date of filing: 13.08.85

(51) Int. Cl.⁴: **A 61 K 37/02,** C 12 P 21/00, C 12 P 21/02

(30) Priority: 13.08.84 GB 8420535
19.10.84 GB 8426549

(43) Date of publication of application: 16.04.86
Bulletin 86/16

(84) Designated Contracting States: BE CH DE FR GB IT LI NL SE

(71) Applicant: THE WELLCOME FOUNDATION LIMITED, 183-193 Euston Road, London NW1 2BP (GB)

(72) Inventor: Kull, Frederick C., Jr., 506 Brookwood Drive, Durham North Carolina 27707 (US)

(74) Representative: Wilkinson, Stephen John et al, c/o Stevens, Hewlett & Perkins 5 Quality Court Chancery Lane, London WC2A 1HZ (GB)

(54) Proteinaceous substance.

(57) A proteinaceous substance which may be isolated from supernatants of stimulated murine macrophage-like cells has potent cytotoxic and in vivo tumor necrotizing activities. The substance comprises a self-aggregate of 15 000 dalton molecular weight protein subunit having an isoelectric point of 4.6 ± 0.3.

EP 0 178 050 A1

## PROTEINACEOUS SUBSTANCE

The present invention relates to a proteinaceous substance having cytotoxic and tissue necrotizing properties, to the isolation and purification of such a proteinaceous substance and to the use of such a proteinaceous substance in the treatment of tumors.

There have been numerous reports on the existence of substances present in the supernatants of monocytes, macrophages and macrophage-like cell lines which substances have been shown to possess cytotoxic activity. Rat peritoneal cells activated by the administration of endotoxin were shown to release a substance cytotoxic to cells in culture, which substance was identified as arginase (Currie, G.A., Nature 273: 758-759, 1978). Other apparently unrelated cytotoxic substances found in supernatants include neutral proteases (Adams, D.O., et al., J. Immunol. 124: 293, 1980) and peroxides (Nathan, C.F., et al., J. Exp. Med. 149: 100, 1979). However, no in vivo antitumor activity has been established for these substances.

It was discovered that the serum of mice infected with bacillus Calmette-Guerin (BCG) and subsequently treated with endotoxin possesses tumor necrotizing and cytotoxic activities (Carswell, E.A., et al., Proc. Nat. Acad. Sci., USA 72: 3666-3670, 1975). This serum is called tumor necrosis serum. These activities were attributed to a "tumor necrosis factor" (TNF) by the authors. The partial purification and preliminary characterization of TNF from mouse serum was carried out by S. Green, et al., Proc. Nat. Acad. Sci., USA 73: 381-385, 1976, by which it was shown that TNF is a glycoprotein containing sialic acid and galactosamine. This glycoprotein has a molecular weight (MW) of approximately 150,000 and has the electrophoretic mobility of α-globulin in cellulose acetate electrophoresis.

Kull, F.C., Jr. and Cuatrecasas, P. (J. Immunol.126: 1279-1283,1981) fractionated tumor necrosis serum by gel filtration. They found that the serum contained multiple forms, e.g., a high MW fraction having MW of 225,000 to 100,000 and a low MW fraction having MW of 50,000. They discovered that a fraction with MW of 160,000 induced tumor necrosis of an intradermal tumor implant in mice, whereas the 225,000 and 50,000 molecular weight fractions induced no tumor necrosis.

We have now isolated a proteinaceous substance from supernatants of stimulated murine macrophage-like cells which proteinaceous substance has potent cytotoxic and in vivo tumor necrotizing activities. While this proteinaceous substance has gross structural features and bioactivities similar to Lymphotoxin, its unique structure may provide distinct therapeutic advantages over other cytotoxic and tumor necrotizing molecules.

The present invention provides a proteinaceous substance comprising a self-aggregate of a protein subunit capable of non-sulfhydryl-linked self-aggregation, which protein subunit has a molecular weight of about 15,000 daltons and an isoelectric point of $4.6 \pm 0.3$. The proteinaceous substance may, in addition to the self-aggregates or multimers of the protein subunit, contain other substances such as binding proteins and/or prosthetic groups.

0178050

A proteinaceous substance according to the invention having potent cytotoxic and in vivo tumor necrotizing activities can be isolated from the serum-free, cell-free supernatants of J774.1 cells (a murine macrophage-like cell line publicly available from the American Type Culture Collection [ATCC], Rockville, MD) stimulated with lipopolysaccharide.

A crude supernatant may be prepared by the following route. The J774.1 cells may be cultured in vitro. Alternatively, the J774.1 cells may be injected intraperitoneally into a host mouse and, after a suitable period of time, the ascites cells harvested from the mouse. The cells produced either in vitro or in vivo are then worked free of extraneous protein, suspended in a medium devoid of extraneous protein, and treated with a lipopolysaccharide, for example lipopolysaccharide from S. typhimurium, and incubated. The crude supernatant is then harvested. The crude supernatant may be subjected to purification techniques, for instance to a combination of ion-exchange and non-denaturing electrophoretic procedures, to give the proteinaceous material in an at least a partially purified form.

In a preferred embodiment, the crude supernatant is concentrated by ultrafiltration and then contacted with an anionic exchange resin. The adsorbed proteinaceous substance is then eluted from the ion-exchange resin and subjected to non-denaturing preparative electrophoresis. The recovered partially purified substance is further applied to an anionic exchange resin.

The substance obtained from this purification procedure, which will hereinafter be referred to as "holotoxin", which typically has a purity of >20%, migrates upon gel filtration in a symmetrical peak as a globular protein with apparent molecular weights of about 70,000 and 55,000 daltons and migrates in a 7.5% non-denaturing polyacrylamide gel with a $R_f$ of 0.7, which is anodal to the majority of supernatant proteins. In addition, the holotoxin on being subjected to sodium dodecyl sulphate (SDS)-polyacrylamide gel electrophoresis migrates with an apparent molecular weight of 15,000 daltons. Elution of this product yields a protein having a molecular weight of 15,000 daltons and a pI of 4.6 ± 0.3. The apparent size of this protein subunit increases to 17,000 daltons upon complete reduction with disulfide reducing agents, which suggests the presence of one or more intramolecular disulfide bond(s).

Accordingly, there is further provided a method of obtaining a cytotoxic material in a substantially pure form, e.g. >95%, which method comprises subjecting the proteinaceous substance of the invention in an at least partially purified form (holotoxin) to SDS-polyacrylamide gel electrophoresis, recovering the material which migrates in a 15,000 molecular weight band in the gel, and then causing the recovered 15,000 molecular weight material to form one or more self-aggregates. Aggregation can be encouraged by concentrating the solution of the purified 15,000 MW material.

By this method, it is possible to obtain a product having a specific activity enriched 8000-fold to a final value of 32,000 units/μg as measured on L-M cells. The material is cytotoxic to other cultured tumorigenic and normal cells in the range of 1-1000 picomolar.

On gel filtration of this self-aggregating 15,000 MW material, the activity peaked sharply and symmetrically in two positions corresponding to molecular weights of 70,000 and 55,000 daltons. In addition, we have detected very small amounts of a protein migrating as a 45,000 dalton species by using an $^{125}$ I radiolabelling technique. Thus, the active forms appear to be dimers, tetramers, and, possibly, trimers of the monomeric form. The aggregates are not sulphydryl-linked. The 15,000 MW material was homogenous since it could not be resolved further by two-dimensional electrophoresis. This 15,000 molecular weight protein accordingly forms a further aspect of the invention. Aggregation of the material appeared to be necessary for cytotoxic activity since the subunit itself was not cytotoxic. Self-aggregates of the 15,000 dalton subunit possessed potent in vivo tumor necrotizing activity. Two picomoles of the aggregate injected intravenously into intradermal fibrasarcoma-bearing mice induced rapid hemorrhagic necrosis within the tumor. We do not know if the non-aggreated 15,000 MW protein possesses necrotizing activity, since this material may undergo aggregation after being introduced into the living system.

Tumor necrosis serum (see J. Immunol. 26: 1279-1283, 1981) and the supernatants from a murine macrophage-like cell line designated RAW 264.7 (publicly available from ATCC) stimulated with lipopolysaccharide (Interleukins, Lymphokines and Cytokines, Academic Press, 1983, pp 511-520) appear to contain a number of cytotoxins by electrophoretic and/or gel filtration procedures. We have compared these with the holotoxin isolated from J774.1 supernatants.

We prepared rat antiserum to the 70,000 dalton holotoxin isolated from J774.1 supernatants in the following way. Female Brown Norway rats were injected intramuscularly in both hind calves with 0.26 ml/site of 20 μg holotoxin emulsified in 50% v/v Freund's complete adjuvant. They were subsequently injected subcutaneously subscapularly at monthly intervals with 10-20 μg holotoxin emulsified in Freund's incomplete adjuvant. Antisera were obtained from the rats 10 days after the third immunization. The antiserum immunoprecipitated the cytotoxic activity of the holotoxin in a concentration-dependent manner. The antiserum also immunoprecipitated activity from the tumor necrosis serum and from the supernatants of stimulated RAW 264.7 cells in an identical manner. Antisera raised in rabbits to the highly purified toxin had the following additional characteristics: one µl completely immunoprecipitated 1000 units of the cytotoxic activity from the murine macrophage-like cell-lines, and it completely neutralised 4000 cytotoxic units in the bioassay. The antisera passively negated the in vivo necrotizing activity of the tumor necrosis serum although preimmune sera had no effect. Thus, the cytotoxic substances tested were all related immunochemically. When the cytotoxic substances from the tumor necrosis serum and from the supernatants of stimulated RAW 264.7 cells were each subjected to SDS-polyacrylamide gel electrophoresis, all the recoverable activity was detected in the 15,000 molecular weight region. In summary, all the cytotoxic substances tested contained the protein subunit having a molecular weight of 15,000 daltons and pI of 4.6 ± 0.3.

The proteinaceous substance of the present invention has some properties similar to those reported for lymphotoxin, a glycosylated protein derived from lymphoid cells. Granger, G.A., et al, Biology of the Lymphokines,1979, Academic Press, Inc, pp 141-163, described the size heterogeneity of human lymphotoxins and classified numerous subtypes into two broad groups: those whose polyacrylamide gel electrophoretic migration was similar to  γ-globulin, and those whose migration was similar to albumin. Aggarwal, B.B., et al; J. Biol.Chem.259: 686-691, 1984 report that human lymphotoxin appears to be composed of aggregates of smaller units. It migrates upon gel filtration with an apparent
MW of 67,000 and upon SDS-gel electrophoresis with an
apparent MW of 20,000. We have tested the
proteinaceous substance of the present invention with
anti-lymphotoxin antiserum. The antiserum did not,
however, neutralize or immunoprecipitate the
proteinaceous substance thus proving that the latter substance and lymphotoxin are not related immunochemically. Furthermore, the amino acid composition of the proteinaceous substance we describe is dramatically different from the composition described for lymphotoxin (Aggarwal, op. cit.). Thus, the two substances share some structural (oligomeric size) and functional (cytotoxic) charcteristics, but their composition is distinct. Their unique activities are likely to reflect these distinctions. For example the two materials are alleged to possess different spectra of toxicity on cell lines (Williamson, B.D., et al., Proc. Natl. Acad. Sci. USA 80: 5397-5401, 1983).

There is yet further provided a method of treating a tumor-bearing host to bring about tumor necrosis which method comprises administering to the host a tumor necrotizing dosage of the proteinaceous substance of the invention. Also provided is a composition for use in such a method of treatment which composition comprises the proteinaceous substance of the invention together with a pharmaceutically acceptable carrier or diluent.

### EXAMPLE

J774.1 cells were grown up in culture. $3 \times 10^6$ washed cells were injected intraperitoneally per BALB/c mouse. Ascites cells were harvested three weeks later. Mice yielded $1-2 \times 10^8$ tumor cells/mouse. The cells were washed first in RPMI 1640, then in cold 0.87% $NH_4Cl$, and again in RPMI 1640. They were suspended at a concentration of $1.2 \times 10^9/L$ in RPMI 1640 which contained 50 µg/ml garamycin and 1 µg/ml of lipopolysaccharide obtained from S. typhimurium LT-2. The suspension was incubated at 37°C for 7 hr after which the supernatant was harvested. Supernatants contained 20-40 mg protein and $1-2 \times 10^5$ cytotoxic units per liter. Lots of 1-2 L of the supernatants were made up to 0.01% sodium azide and 50 µM phenylmethylsulfonylfluoride. The supernatant was centrifuged at 1500 g to remove particulate material and 500 ml of supernatant were then concentrated 100- to 200-fold by ultrafiltration versus a PM 30 membrane (a 30,000 MW pore size membrane from Amicon). The concentrate was dialyzed versus equilibrating buffer (50 mM Tris-HCl, pH 8.0, 15 mM NaCl) and applied to a Mono Q anion exchange column (a hydrophilic polymer containing quaternary amino groups, obtained from Pharmacia). 1 ml fractions/minute were collected. Activity was eluted with a linear 15-500 mM NaCl gradient. The active fractions were pooled and reapplied to the column. Elution was then carried out with a 100-300 mM NaCl gradient. The active fractions were pooled and concentrated to 1 ml by ultrafiltration versus a PM 10 membrane (a 10,000 MW pore size membrane from Amicon). The concentrate was diluted 10-fold with H₂0, reconcentrated and applied to preparative electrophoresis in a 1100 PG device (Bethesda Research Laboratories) employing a 1 x 5 cylindrical 7.5% polyacrylamide gel (gel system of Laemmli, U.K., Nature 227: 680-685, 1970, but devoid of SDS and a stacking gel). The electrophoresis was performed in the cold with a constant current of 5 mamps.

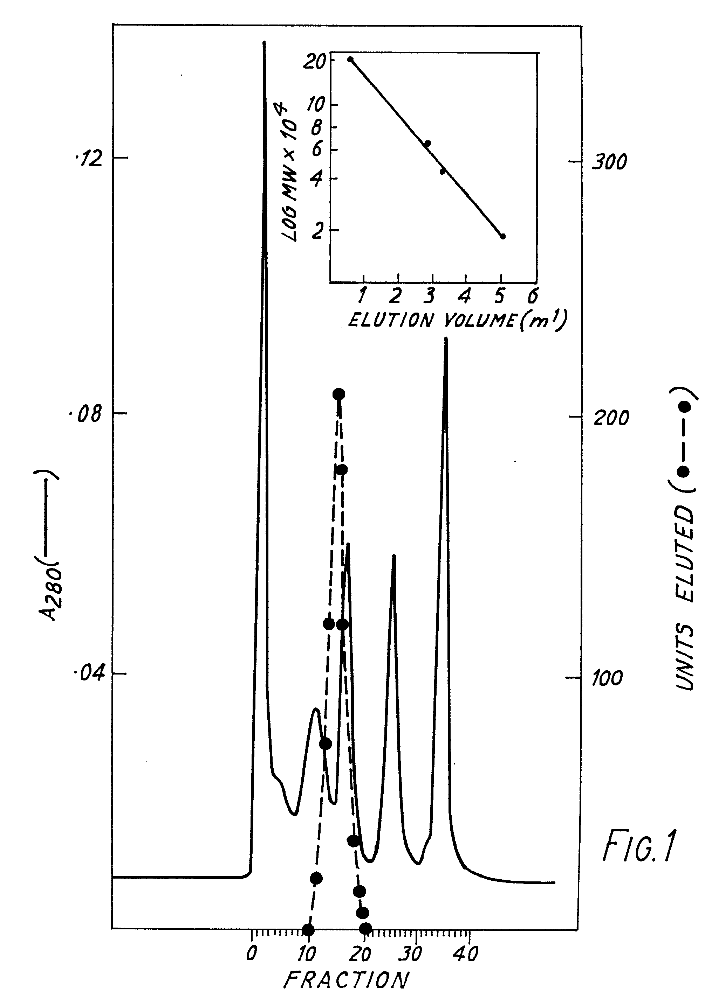

The elution rate was 1.1 ml/fraction/9 min. Active fractions were pooled, diluted with equilibrating buffer, concentrated by ultrafiltration and applied to a Mono Q anion exchange column. Activity was eluted with a linear 15-500 mM NaCl gradient and the active fractions were collected and pooled. The apparent size of the holotoxin was determined by gel filtration as described below. 900 Units (150 ng) of the holotoxin obtained/10 µl was mixed with 5 µl of a sizing standards preparation (Biorad). The mixture was applied to a HPLC system (Waters Associates, Morristown, New Jersey), fitted with a TSK 250 gel filtration column (Biorad) run in 150 mM NaCl, 20 mM NaP$_i$ (sodium phosphate), pH 7.4. The flow rate was 1 ml/min, and 0.2 ml fractions were collected and assessed for toxicity. The results are shown in Fig. 1. In fact, the A$_{280}$ of the holotoxin was too low for detection. The four major peaks in Figure 1 (solid line) show the standards: thyroglobulin (molecular weight 669,000 daltons); ovalbumin (molecular weight 45,000 daltons); myoglobulin (molecular weight 18,000 daltons); and phenylalanine (molecular weight 165). Ovalbumin and myoglobin gave a linear log molecular weight versus elution volume relationship from which the molecular weight of the holotoxin was determined to be about 70,000 daltons.

The holotoxin was subjected to further purification as follows.
The pooled fractions obtained from the previous anionic exchange
resin treatment were subjected to SDS-polyacrylamide gel electrophoresis using the system of Laemmli, U.K., Nature 227: 680-685,
1970, but devoid of a stacking gel and using an SE 600 slab gel
apparatus (Hoeffer Scientific Instruments). For example, holotoxin
(180 ng/1000 units) was applied to a lane in a 140 x 0.75 mm slab
gel containing 0.1% SDS, 15% polyacrylamide and 0.25 crosslinker.
The results of the SDS-polyacrylamide gel electrophoresis are
illustrated in Figure 2. The left lane is holotoxin and the right
line contains a number of molecular weight protein standards
(obtained from Bethesda Research Laboratories, Bethesda, Maryland).
The standards are from top to bottom (major bands): phosphorylase
B, MW 92,500; bovine serum albumin, MW 66,200; ovalbumin, MW 45,000;
carbonic anhydrase, MW 31,000; soybean trypsin inhibitor, MW 21,500;
lysozyme, MW 14,400. An identical holotoxin lane was sliced
(2.23 mm/slice). The slices were eluted overnight by soaking in
200 ul 150 mM NaCl, 20 mM NaPi, pH 7.4 and 0.1% bovine serum
albumin to collect the purified subunit protein. The graph in
Figure 2, which shows the corresponding position of the gel slices
and the units eluted (note the logarithmic scale), illustrates
that the holotoxin migrates, on SDS-polyacrylamide gel electrophoresis,
with an apparent MW of 15,000. A small amount migrates with an
apparent MW of 45,000. Larger quantities of the 15,000 dalton
protein were collected similarly, except that the holotoxin was
layered preparatively across the entire gel slab, and the region
containing the 15,000 dalton protein was eluted electrophoretically according to the method described by Hunkapillar, M.W., et al., Met. Enzymol. 91: 227-236, 1983, except without SDS and without a presoak in dithiothreitol. In this manner, µg quantities of the purified toxin were obtained. The toxin constituted better than 95 percent of the total protein in the electrophoretic eluant as assessed by photometric scanning of silver stained SDS-polyacrylamide gels.

Gel filtration was performed on the purified toxin in a manner identical to that performed on the holotoxin as described above, and dilutions were measured for cytotoxic activity. The activity was found to peak sharply and symmetrically in a position corresponding to MW of 70,000. A second lot eluted with a MW of 55,000.

The stepwise purification is given in Table 1 below.

Table I. <u>Purification of toxin</u>.

| Step | Units | Protein (mg) | Specific Activity (U/µg) | Fold Purification | Yield (%) |
|---|---|---|---|---|---|
| Supernatant | 960,000 | 240 | 4 | - | 100 |
| Concentrate | 960,000 | 80 | 12 | 3 | 100 |
| Anion Exchange | 720,000 | 1.8 | 400 | 100 | 75 |
| Non SDS Preparative PAGE | 450,000 | 0.09 | 5,000 | 1,250 | 47 |
| Anion Exchange | 360,000 | 0.06 | 6,000 | 1,500 | 38 |
| SDS PAGE, Electrophoretic Elution | 320,000 | 0.01 | 32,000 | 8,000 | 33 |

## Toxicity

### In vitro toxicity

An _in vitro_ study of the cytotoxicity of the isolated holotoxin on a number of cell lines was carried out according to the method of Kull, F.C., Jr. and Cuatrecasas P. (Applied Biochem. Biotech. **8**: 97-103, 1983). The cell lines tested were the following: the L-M line (ATCC CCL 1.2), a murine tumorigenic fibroblast; the CPAE line (ATCC CCL 209), bovine aortic endothelial cells; $MCF_7$ (ATCC HTB 22), a human mammary carcinoma line; and Hep $G_2$ (ATCC HB 8065), a human liver carcinoma line.

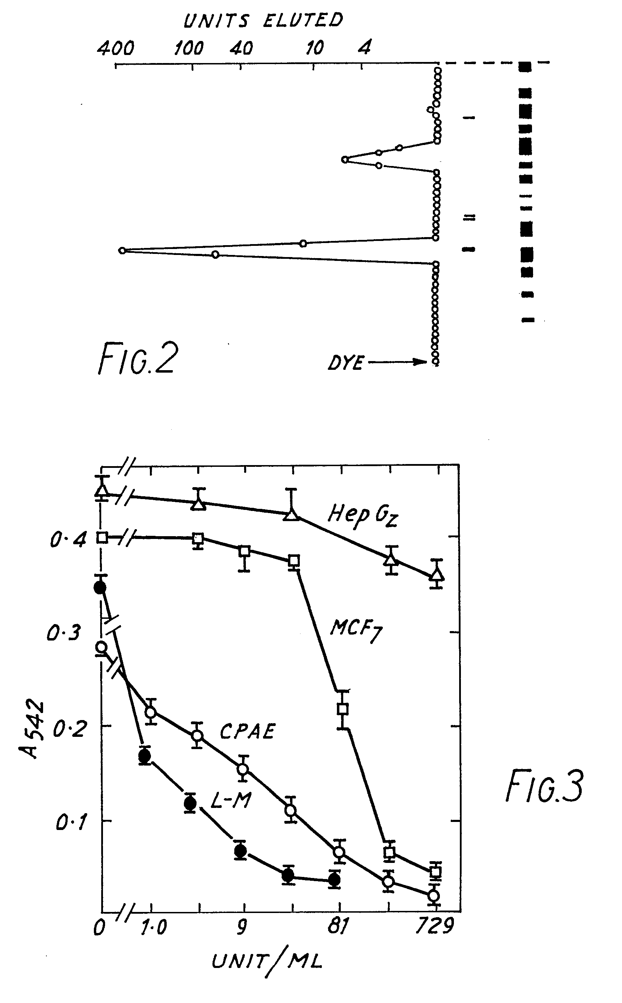

The cell lines were seeded in their usual growth media in microtiter trays, roughly 5000-7500 cells/well/200 µl medium, and incubated overnight. $Log_3$ dilutions of holotoxin were added the following day. The cell lines were left to incubate until toxicity was evident by light microscopic examination. The trays were then scored with neutral red according to Kull, F.C.. Jr. and P. Cuatrecasas, Applied Biochem. Biotech. **8**: 97-103, 1983. Cell lines differ in their capacity to internalize neutral red (control $A_{542}$); however, the curve for each cell line, shown in Figure 3 gives a relative indication of the number of living cells. All of the cell lines tested were sensitive; the L-M cell line was the most sensitive.

Dose response of toxin on _in vivo_ tumor necrosis

---

Intradermal implants were prepared using a methyl-cholanthrene-induced transplantable fibrosarcoma in BALB/c mice. One week later, when the tumor diameter was approximately 5 mm, mice received a single 0.5 ml intravenous injection (i.v.) of test holotoxin which had been dialyzed or diluted in pyrogen-free saline. Control animals received 0.5 ml filtrate (dialysis solution). Animals were scored for tumor necrosis after 24 hr, where the scale range from no apparent necrosis (0) to necrosis covering the entire diameter of the tumor (+4).

| | Number Mice | | | | |
| --- | --- | --- | --- | --- | --- |
| Cytotoxic Units | Necrosis Score | | | | |
| | 0 | +1 | +2 | +3 | +4 |
| 0 | 6 | | | | |
| 1,000 | 3 | 2 | | | |
| 2,000 | | 2 | 2 | 1 | |
| 4,000 | | | 1 | 2 | 2 |
| 8,000 | | 1 | | 1 | 3 |

From the results tabulated above, it is clear that the holoxotin contains the capacity to induce necrosis of an intradermal implant. The tumor necrotizing capacity was dose dependent.

Additional trials have confirmed that 4000-8000 units reproducibly induced substantial tumor necrosis in test mice. Necrosis was visibly apparent (redness of the tumor) within 2 hr of injection. We found that the effective but sub-lethal necrotizing dose range was 4000-12000 units.

The preparation was examined for the presence of endotoxin since this substance also possesses this activity and because LPS was a component of the crude starting material.

Although 4000 units (500 ng protein) of toxin gave substantial necrosis as can be seen above, it contained 1000 times less endotoxin as determined by the Limulus amebocyte lysate assay than that amount of LPS alone necessary to produce necrosis.

The purified toxin contained necrotizing activity. Holotoxin was fractionated by SDS-preparative electrophoresis. The major bands were excised and isolated by electrophoretic elution as described in the example. The material obtained from the 15,000 MW band was diluted 20-fold in pyrogen-free saline to 4000 units (125 ng/0.5 ml). The materials obtained from the other bands were similarly diluted. All were tested for necrotizing activity. Necrosis was observed only in the group of mice which received the material obtained from the 15,000 MW band.

Aggregation of subunits is necessary for cytotoxic activity on L-M cells. Assuming a molecular weight of 70,000 for the purified toxin (i.e., the reaggregated material), the concentration of toxin which produced one cytotoxic unit on L-M cells was

$2 \times 10^{-12}$ M.  The dose of the purified toxin required to induce necrosis of an intradermal tumor was 125 ng (2 nmole) given i.v.

A single dose of 500 ng i.v. was lethal to tumor-bearing mice.

## CLAIMS

1.      A proteinaceous material having a molecular weight of about 15,000 daltons as measured by SDS-polyacrylamide gel electrophoresis and an isoelectric point of 4.6 ± 0.3 and which is capable of non-sulfhydryl-linked self-aggregation to form self-aggregates having antitumour activity.

2.      A proteinaceous substance in a substantially pure form and having antitumour activity which comprises a self-aggregate of a protein subunit capable of non-sulfhydryl-linked self-aggregation which protein subunit has a molecular weight of about 15,000 daltons as measured by SDS-polyacrylamide gel electrophoresis and an isoelectric point of 4.6 ± 0.3.

3.      A proteinaceous substance according to claim 2, which, in addition to the self-aggregate of the protein subunit, may contain one or more binding proteins and/or prosthetic groups.

4.      A proteinaceous substance according to claim 3, having a molecular weight of about 70,000 daltons as measured by gel filtration.

5.      A proteinaceous substance according to claim 3, having a molecular weight of about 45,000 - 55,000 daltons as measured by gel filtration.

6.      A proteinaceous substance according to either claim 4 or claim 5, which migrates in a 7.5% non-denaturing polyacrylamide gel with a $R_f$ of 0.7.

7.      A proteinaceous substance according to claim 2, which has a specific cytotoxic activity against L-M cells of about $3.2 \times 10^7$ units/mg.

8.      A proteinaceous substance according to claim 2 which is of murine origin.

9.          A method of preparing the proteinaceous substance of claim 2 comprising subjecting the proteinaceous substance in a partially purified form to SDS-polyacrylamide gel electrophoresis, recovering material from a band in the gel corresponding to a molecular weight of about 15,000 daltons and then causing the recovered material to self-aggregate.

10.         A method according to claim 9, wherein the proteinaceous substance in a partially purified form is obtained by subjecting a crude solution of the proteinaceous substance to ultrafiltration, contacting with a first anionic exchange resin, eluting the adsorbed substance from the ion-exchange resin and subjecting to non-denaturing preparative electrophoresis followed by contacting the recovered substance with a second anionic exchange resin and eluting the adsorbate.

11.         A method according to claim 10, wherein the crude solution of the proteinaceous substance comprises body fluids, serum, plasma or extract of internal organs of a mammal to which had previously been administered a substance capable of stimulating the reticuloendothelial system followed by an injecting of endotoxin.

12.         A method according to claim 10, wherein the crude solution of the proteinaceous substance comprises a serum-free supernatant collected from a tissue culture of macrophage-like cells which has been treated with a lipopolysaccharide and incubated.

13.         A method according to claim 11, wherein the tissue culture is a culture of murine macrophage-like cells.

14.         A pharmaceutical composition for use in the in vivo treatment of tumours which composition comprises the proteinaceous substance of any one of claims 2 to 8 in a tumor necrotizing dosage together with a pharmaceutically acceptable carrier or diluent.

15.       A method of treating a tumor-bearing host to bring about tumor necrosis comprising administering to the host a tumor necrotizing dosage of the proteinaceous substance of any one of claims 2 to 8.

16.       Any portion of the proteinaceous substance according to claims 1 to 7 as deduced from its structure which shares the bioactivity of the parent substance.

FIG.1

0178050

UNITS ELUTED

400    100    40    10    4

FIG.2

DYE ⟶

FIG.3

*A542*

HepGZ

MCF7

CPAE

L-M

0·4

0·3

0·2

0·1

0    1·0    9    81    729

UNIT/ML

## PARTIAL EUROPEAN SEARCH REPORT

European Patent Office

which under Rule 45 of the European Patent Convention shall be considered, for the purposes of subsequent proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A- 0 100 641 (GENENTECH INC.)<br><br>* Claims; page 4, last paragraph - page 5, line 3; page 7, lines 1-7; page 11, line 12; page 13, last sentence; page 14, lines 12-17; page 17, lines 9-11 *<br><br>--- | 1-14, 16 | C 07 K 15/00<br>C 07 K 3/12<br>A 61 K 37/02//<br>C 12 P 21/00 |
| A | INFECTION AND IMMUNITY, vol. 28, no. 1, April 1980, pages 204-211; D.N. MÄNNEL et al.: "Generation and characterization of a lipopoly-sacharide-induced and serum-derived cytotoxic factor for tumor cells."<br><br>* Page 208, right-hand column; page 210, left-hand column, line 27 - end *<br><br>--- | 1-14, 16 | |
| A | INFECTION AND IMMUNITY, vol. 30, no. 2, November 1980, pages 523-530;<br><br>./. | | TECHNICAL FIELDS SEARCHED (Int. Cl.4)<br><br>C 07 K<br>A 61 K<br>C 12 P<br>C 12 N |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-14,16

Claims searched incompletely:

Claim not searched: 15

Reason for the limitation of the search:

Method for treatment of the human or animal body by surgery or therapy; see article 52(4) of the European Patent Convention.

| Place of search | Date of completion of the search | Examiner |
|---|---|---|
| The Hague | 26-11-1985 | RIJCKEBOSCH |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82

European Patent
Office

**PARTIAL EUROPEAN SEARCH REPORT**

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | D.N. MÄNNEL et al.: "Macrophages as a source of tumoricidal activity (tumor necrotizing factor)." | | |
| | * Page 527, left-hand column, line 22 - right-hand column, line 13 * | 1-14, 16 | |
| | --- | | |
| A | THE JOURNAL OF IMMUNOLOGY, vol. 122, no. 5, May 1979, pages 1785-1790; R.R. AKSAMIT et al.: "Microphage cell lines produce a cytotoxin." | | |
| | * Page 1787, right-hand column, table IV * | 1-14, 16 | TECHNICAL FIELDS SEARCHED (Int. Cl.4) |
| | --- | | |
| A | THE JOURNAL OF IMMUNOLOGY, vol. 125, no. 4, October 1980, pages 1671-1677; M.R. RUFF et al.: "Purification and physico-chemical characterization of rabbit tumor necrosis factor." | | |
| | * Page 1676, left-hand column, lines 24-50 * | 1-14, 16 | |
| | --- | | |
| A | CHEMICAL ABSTRACTS, vol. 99, no. 17, 24th October 1983, page 445, abstract 137978p, Columbus, Ohio, US H. FISCH et al.: "In vitro production of rabbit macrophage tumor cell cytotoxin." & INT. J. CANCER, 1983, 31(1), 105-12 | | |
| | * The whole abstract * | 1-14, 16 | |
| | --- | | |
| A | EP-A- 0 086 475 (NIPPON SHINYAKU CO. LTD.) | | |
| | * Claims; page 6, lines 19-25 * | 1-14, 16 | |
| | --- | | |
| | ./. | | |

EPO Form 1505.3 06.78

## European Patent Office

## PARTIAL EUROPEAN SEARCH REPORT

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | EP-A- 0 090 892 (ASAHI KASEI KOGYO K.K.) <br><br> * Claims * | 1-14, 16 | |
| | ---------- | | |
| | | | TECHNICAL FIELDS SEARCHED (Int. Cl.4) |

EPO Form 1505.3  06.78